# EUROPEAN PATENT APPLICATION

(11) **EP 1 640 719 A1**
(43) Date of publication of application: **29.03.2006**
(21) Application number: 04736582.0
(22) Date of filing: 10.06.2004
(51) Int. Cl.: G01N 33/53

(54) **METHOD OF ADJUDICATING ON ACUTE AORTIC DISSECTION AND REAGENT FOR ADJUDICATION**

(30) Priority: 10.06.2003 JP 2003165456
(71) Applicant: Dainippon Sumitomo Pharma Co., Ltd., Osaka 541-8524 (JP)
(72) Inventor: HAZUI, Hiroshi, Mino-shi, Osaka 5620022 (JP); NISHIMOTO, Masayoshi, Mezonkureru 301, Nada-ku, Kobe-shi, Hyogo 6570054 (JP); TAKESHITA, Hitoshi, Arutipurazayasu 607,665-1, Yasu-gun, Shiga 5202352 (JP); OHKARU, Yasuhiko, Sennan-gun, Osaka 5990301 (JP)
(74) Representative: Duckett, Anthony Joseph
(86) International application number: PCT/JP2004/008471
(87) International publication number: WO 2004/111645

(57) **Abstract**

A method of evaluating acute aortic dissection, which comprises measuring a D-dimer concentration in blood separated from a human, and determining whether or not acute aortic dissection (Stanford type A or Stanford type B) has developed, on the basis of the measured concentration, a reagent for evaluating performing the evaluation method, and the like are provided.

## Description

### Technical Field

The present invention relates to a method of evaluating acute aortic dissection, which comprises measuring a D-dimer concentration in blood separated from a human, and determining whether or not acute aortic dissection has developed, on the basis of the measured concentration, and the like.

The present invention also relates to a reagent for evaluating acute aortic dissection, which comprises an antibody that recognizes a D-dimer (hereinafter also referred to as "anti-D-dimer antibody"), and which is used to perform the above-described evaluation method, and the like.

### Background Art

The incidence of acute aortic dissection is higher than generally considered, and it ranks second to acute myocardial infarction in terms of mortality among cases of sudden death due to cardiovascular disease.

This disease is characterized by violent chest pain as the chief complaint and sudden onset occasionally followed by the outcome of death in a short time; for appropriate procedure and treatment, it is critical importance to distinguish this disease from acute myocardial infarction, pulmonary infarction, threatened rupture of thoracic aneurysm, pneumothorax and the like, which are also characterized by chest pain as the chief complaint.

In particular, in Stanford type A acute aortic dissection, which is described below, dissociation sometimes involves the coronary artery and occludes the vessels, which in turn causes the pathologic condition of acute myocardial infarction. If a patient with acute aortic dissection is misdiagnosed and undergoes thrombolytic therapy, which is a treatment for acute myocardial infarction, the patient can die due to massive bleeding from the aorta.

Hence, in medical settings, particularly in emergency medical settings, acute aortic dissection is deemed a disease with violent chest pain as the chief complaint, which is particularly difficult to distinguish from acute myocardial infarction.

D-dimer is a protein within an organism, which is utilized to extensively assess the pathologic condition of hyperactivity of the blood coagulation/fibrinolytic system as a marker for hyperactivity of the secondary fibrinolytic system. Specifically, it is used as a marker for diagnosis, pathologic assessments, and therapeutic effect evaluation in disseminated intravascular coagulation (DIC) and various thrombotic diseases.

In the Journal of the Japanese Association for Thoracic Surgery, July 10, 1998, Vol. 46, No. 7, pp. 616-621, it is reported that preoperative and postoperative hemoglobin (Hb) levels, platelet counts, fibrinogen levels, antithrombin (AT) III levels, prothrombin times, activated partial thromboplastin times, FDP levels and D-dimer levels were measured as blood coagulation parameters, so as to examine the hemostatic effect of high-dose administration of tranexamic acid (hemostatic agent) in surgery for patients with acute aortic dissection.

However, in the aforementioned reference, the D-dimer was measured only as a blood coagulation parameter for examining the effect of high-dose administration of tranexamic acid in surgery for patients with acute aortic dissection.

Also, in CHEST (USA), May 2003, Vol. 123, No. 5, pp. 1375-1378, it is described that the mean D-dimer concentration in blood from 24 patients with acute aortic dissection was 9.4 µg/mL.

In the invention of this application, as shown in an Example below, the mean D-dimer concentration in blood from patients with acute aortic dissection was 33.0 µg/mL, showing a wide difference from the value given in the aforementioned reference.

Additionally, in these two references, there is no description or suggestion about the fact that Stanford type A acute aortic dissection and Stanford type B acute aortic dissection can be distinguished from each other on the basis of D-dimer concentration in blood.

### Disclosure of Invention

An object of the present invention is to provide a evaluation method and reagent for evaluating acute aortic dissection, and the like.

The present inventors drew blood from patients developing acute aortic dissection, and measured a D-dimer concentrations therein. As a result, the present inventors found that much higher concentrations of D-dimer are present in patients with acute aortic dissection than in patients with acute myocardial infarction, who are known to have higher concentrations of D-dimer in their blood than healthy people, and that the concentrations differ widely between Stanford type A acute aortic dissection and Stanford type B acute aortic dissection, which are described below, and completed the present invention.

Accordingly, the present invention provides:
[1] a method of evaluating acute aortic dissection, which comprises measuring a D-dimer concentration in blood separated from a human, and determining whether or not acute aortic dissection has developed, on the basis of the measured concentration,
[2] a method of evaluating acute aortic dissection, which comprises measuring a D-dimer concentration in blood separated from a human, and determining whether or not Stanford type A acute aortic dissection has developed, on the basis of the measured concentration,
[3] a method of evaluating acute aortic dissection, which comprises measuring a D-dimer concentration in blood separated from a human, and determining whether or not Stanford type B acute aortic dissection has developed, on the basis of the measured concentration,
[4] a method of evaluating acute aortic dissection, which comprises measuring a D-dimer concentration in blood separated from a human developing acute aortic dissection, and determining whether the developed acute aortic dissection is Stanford type A acute aortic dissection or Stanford type B acute aortic dissection, on the basis of the measured concentration,
[5] a method of distinguishing between acute aortic dissection and acute myocardial infarction, which comprises measuring a D-dimer concentration in blood separated from a human having an episode of chest pain, and determining which disease has developed whether acute aortic dissection or acute myocardial infarction, on the basis of the measured concentration,
[6] the distinguishing method described in [5] above, which comprises comparing the measured D-dimer concentration in blood and a D-dimer cutoff value in blood which is pre-established between acute aortic dissection and acute myocardial infarction, and determining that acute aortic dissection has developed if said concentration is not lower than said cutoff value, and acute myocardial infarction has developed if said concentration is lower than said cutoff value,
[7] a method of distinguishing between acute aortic dissection and acute myocardial infarction, which comprises measuring a D-dimer concentration in blood separated from a human having an episode of chest pain, and determining which disease has developed whether Stanford type A acute aortic dissection, Stanford type B acute aortic dissection, or acute myocardial infarction, on the basis of the measured concentration,
[8] the evaluation method for acute aortic dissection described in any of [1] to [4] above, wherein the measurement of D-dimer concentration in blood is performed by an immunochemical method,
[9] the evaluation method described in [8] above, wherein the immunochemical method is an enzyme immunochemical method, a latex aggregation method, or an immunochromatography method,
[10] the method of distinguishing between acute aortic dissection and acute myocardial infarction described in any of [5] to [7] above, wherein the measurement of D-dimer concentration in blood is performed by an immunochemical method,
[11] the distinguishing method described in [10] above, wherein the immunochemical method is an enzyme immunochemical method, a latex aggregation method, or an immunochromatography method,
[12] a reagent for evaluating acute aortic dissection, which comprises an antibody that recognizes a D-dimer,
[13] the evaluation reagent described in [12] above, which is a reagent for evaluating Stanford type A acute aortic dissection,
[14] the evaluation reagent described in [12] above, which is a reagent for evaluating Stanford type B acute aortic dissection,
[15] a reagent for distinguishing between Stanford type A acute aortic dissection and Stanford type B acute aortic dissection, which comprises an antibody that recognizes a D-dimer,
[16] the reagent described in any of [12] to [15] above, wherein the antibody is a monoclonal antibody,
[17] a reagent for distinguishing between acute aortic dissection and acute myocardial infarction, which comprises an antibody that recognizes a D-dimer,
[18] the distinguishing reagent described in [17] above, which is a reagent for distinguishing between Stanford type A acute aortic dissection and acute myocardial infarction,
[19] the distinguishing reagent described in [17] above, which is a reagent for distinguishing between Stanford type B acute aortic dissection and acute myocardial infarction,
[20] the distinguishing reagent described in [17] above, which is a reagent for distinguishing among Stanford type A acute aortic dissection, Stanford type B acute aortic dissection, and acute myocardial infarction,
[21] the distinguishing reagent described in any of [17] to [20] above, wherein the antibody is a monoclonal antibody,
[22] a commercial package comprising the reagent described in any of [12] to [15] above and a printed matter on the reagent, wherein the printed matter and/or the package bears the statement that the reagent can be used, or should be used, for the purpose of evaluating acute aortic dissection,
[23] a commercial package comprising the distinguishing reagent described in any of [17] to [20] above and a printed matter on the reagent, wherein the printed matter and/or the package bears the statement that the reagent can be used, or should be used, for the purpose of distinguishing between acute aortic dissection and acute myocardial infarction,
[24] use of an antibody that recognizes a D-dimer, for producing a reagent for evaluating acute aortic dissection,
[25] use of an antibody that recognizes a D-dimer, for producing a reagent for distinguishing between acute aortic dissection and acute myocardial infarction,
[26] a method of evaluating acute aortic dissection, which comprises detecting a D-dimer in blood separated from a human with suspected acute aortic dissection,
[27] the evaluation method described in [26] above, wherein the human with suspected acute aortic dissection is a human having an episode of chest pain,
[28] the evaluation method described in [26] or [27] above, wherein D-dimer detection is performed by an immunochemical method using an antibody that recognizes the D-dimer,
[29] the evaluation method described in [28] above, wherein the immunochemical method is an enzyme immunochemical method, a latex aggregation method, or an immunochromatography method,
[30] a method of evaluating whether acute aortic dissection or acute myocardial infarction has developed, which comprises detecting a D-dimer in blood separated from a human with suspected acute aortic dissection and/or acute myocardial infarction,
[31] the evaluation method described in [30] above, wherein the human with suspected acute aortic dissection and/or acute myocardial infarction is a human having an episode of chest pain,
[32] the evaluation method described in [30] or [31] above, which comprises comparing the detected D-dimer concentration in blood and a D-dimer cutoff value in blood which enables distinguishing between acute aortic dissection and acute myocardial infarction, and determining that acute aortic dissection has developed if said concentration is not lower than the said cutoff value, and that acute myocardial infarction has developed if said concentration is lower than said cutoff value,
[33] the evaluation method described in any of [30] to
[32] above, wherein D-dimer detection is performed by an immunochemical method using an antibody that recognizes the D-dimer,
[34] the evaluation method described in [33] above, wherein the immunochemical method is an enzyme immunochemical method, a latex aggregation method, or an immunochromatography method,
[35] the evaluation method described in any of [26] to [34] above, wherein the acute aortic dissection is acute aortic dissection classified as type A according to the Stanford Classification,
[36] the evaluation method described in any of [26] to [34] above, wherein the acute aortic dissection is acute aortic dissection classified as type B according to the Stanford Classification,
[37] a reagent for evaluating acute aortic dissection, which is used to perform the evaluation method described in [28] or [29] above, and which comprises at least an antibody that recognizes the D-dimer,
[38] a commercial package comprising the evaluation reagent described in [37] above, which has the statement thereon that the evaluation reagent can be used, or should be used, for the purpose of evaluating acute aortic dissection, or comprises a printed matter on the evaluation reagent bearing the same statement therein,
[39] a reagent for evaluating whether acute aortic dissection or acute myocardial infarction has developed, which is used to perform the evaluation method described in [33] or [34] above, and which comprises at least an antibody that recognizes a D-dimer,
[40] a commercial package comprising the evaluation reagent described in [39] above, which has the statement thereon that the evaluation reagent can be used, or should be used, for the purpose of distinguishing between acute aortic dissection and acute myocardial infarction, or comprises a printed matter on the evaluation reagent bearing the same statement therein,
[41] the evaluation reagent or commercial package described in any of [37] to [40] above, wherein the acute aortic dissection is acute aortic dissection classified as type A according to the Stanford Classification, and
[42] the evaluation reagent or commercial package described in any of [37] to [40] above, wherein the acute aortic dissection is acute aortic dissection classified as type B according to the Stanford Classification.

### Brief Description of the Drawings

FIG. 1 shows an ROC curve generated from the D-dimer concentrations in blood from cases with acute aortic dissection and cases with acute myocardial infarction in Example 2.
FIG. 2 shows the distribution of the D-dimer concentrations in blood from cases with acute aortic dissection and cases with acute myocardial infarction in Example 2.
FIG. 3 shows a distribution map of measured values of the D-dimer concentrations in blood from cases with acute aortic dissection (Stanford type A and Stanford type B) and cases with acute myocardial infarction in Example 5.
FIG. 4 shows an ROC curve generated in Example 6.

### Best Mode for Carrying out the Invention

The present invention relates to a method of evaluating acute aortic dissection, which comprises detecting a D-dimer in blood separated from a human, for example, a human with suspected acute aortic dissection. In other words, the present invention relates to a method of evaluating acute aortic dissection, which comprises measuring a D-dimer concentration in blood separated from such a human, and determining whether or not acute aortic dissection has developed, on the basis of the measured concentration.

As used herein, "D-dimer concentration" has the same meaning as "D-dimer level", and "measuring a D-dimer concentration" has the same meaning as "detecting a D-dimer (level)".

Acute aortic dissection, the subject disease for the evaluation in the present invention, is a disease characterized by the pathologic condition wherein the tunica media, which constitutes the aortic wall, is dissociated and cleaved to the intimal side and the adventitial side. Histologically, the aortic wall consists of the tunica intima, the tunica media, and the tunica adventitia in this order from the vascular lumen side.

The typical initial symptom of this disease is suddenly developing violent chest pain, and this symptom is generally called an "episode of chest pain". The pain in an episode of chest pain may not be localized to the chest and may involve the back to show signs of chest back pain, depending on the onset region and size of acute aortic dissection. Note that this pain is generally understood to result from a rupture of the aortic wall.

In this disease, clinical symptoms and therapeutic methods differ depending on the region of onset of dissociation; for this reason, classification of disease types according to the region of onset of dissociation is performed and utilized for deciding a therapeutic policy. According to the representative classification of disease types by Stanford, any case of this disease is classified as either "Stanford type A acute aortic dissection", in which dissociation is present in the ascending aorta from the heart to the neck, or "Stanford type B acute aortic dissection", in which dissociation is not present in the ascending aorta, more specifically dissociation is not present in the ascending aorta but is present in the descending aorta from the thoracic aorta to the abdominal aorta.

Among patients with Stanford type A acute aortic dissection are many patients whose dissociation involves the coronary artery and result in serious complications or arterial rupture; the spontaneous prognosis for Stanford type A acute aortic dissection is much worse than that for Stanford type B acute aortic dissection. Therefore, if it is determined that acute aortic dissection has developed, it is important in the field of emergency medical care to distinguish whether Stanford type A acute aortic dissection or Stanford type B acute aortic dissection has developed.

Due to the above-described typical clinical symptoms in acute aortic dissection, a human having an episode of chest pain is the subject of D-dimer detection in another mode of embodiment of the present invention.

In the present invention, a measurement of D-dimer concentration in blood separated from a human is not subject to limitation; for example, the measurement can be performed by any of immunochemical methods, various chromatographies such as HPLC, and the like. In particular, the immunochemical method utilizing an anti-D-dimer antibody is preferably used to measure the concentration. As used herein, "to measure D-dimer concentration" means not only a quantitative measurement of D-dimer concentration, but also qualitative detection of the presence or absence of D-dimer at a level above a given concentration.

The immunochemical method used to measure D-dimer concentration in blood is not subject to limitation; as examples of conventionally known methods, enzyme immunoassay (EIA method), latex aggregation method, immunochromatography method, radioimmunoassay (RIA method), fluorescence immunoassay (FIA method), luminescence immunoassay, spin immunoassay, nephelometry for judging the turbidity resulting from the formation of antigen-antibody complex, the enzyme sensor electrode method for detecting potential changes by the binding with an antigen using an antibody-immobilized membrane electrode, immune electrophoresis, Western blotting, and the like can be mentioned. Of these, the EIA method, the latex aggregation method or the immunochromatography method is preferably used to measure D-dimer concentration.

The EIA method includes the competitive method, wherein an enzyme-labeled antigen and an antigen in the sample are allowed to compete with each other, and the non-competitive method, which does not involve such competition; of these methods, the sandwich enzyme-linked immunosorbent assay (sandwich ELISA method), a kind of non-competitive method using two kinds of antibodies, especially monoclonal antibodies, is particularly preferable with respect to specificity for antigen (D-dimer) and the ease of detection operation.

The sandwich ELISA method comprises measuring D-dimer concentration by putting (sandwiching) the D-dimer between two kinds of antibodies that recognize different epitopes present in the D-dimer, i.e., a solid-immobilized anti-D-dimer antibody and an enzyme-labeled anti-D-dimer antibody. Hence, D-dimer concentration can be measured by measuring the enzyme amount in the labeled antibody bound to the D-dimer captured by the solid-immobilized antibody.

As a kind of the sandwich ELISA method, a method utilizing the avidin-biotin reaction is also available. According to this method, it is possible to measure D-dimer concentration in the same manner as described above, by capturing the D-dimer in blood with a solid-immobilized anti-D-dimer antibody, allowing an antigen-antibody reaction between the captured D-dimer and a biotin-labeled anti-D-dimer antibody, and then adding enzyme-labeled streptoavidin.

The latex aggregation method is an immunochemical method utilizing the aggregation reaction between antibody-sensitized latex particles and an antigen. A measurement of D-dimer concentration by this method can be performed by allowing an immune reaction between anti-D-dimer antibody-sensitized latex particles and the D-dimer in the sample blood, and measuring the extent of the resulting aggregation of the latex particles.

In addition, the immunochromatography method is a method wherein all immunochemical reaction systems are retained on a sheet-like carrier and the operation is completed only with the addition of blood. The outline of the measurement of D-dimer concentration by this method is as follows. First, when the sample blood is added dropwise to the carrier, an immune reaction takes place between the D-dimer in the blood and an anti-D-dimer antibody labeled with a marker (gold colloid and the like) placed on the carrier, resulting in the formation of an immune complex. This complex develops over the carrier; when it is captured by an anti-D-dimer antibody that recognizes another epitope immobilized on a particular portion on the carrier, the marker accumulates; by macroscopically observing the extent of this accumulation, D-dimer concentration can be measured.

In performing immunochromatography, no special measuring instrumentation is required; this method is advantageous in cases where diseases is evaluated outside the hospital, or immediate evaluation of diseases in emergencies and the like is desired. This method is suitable for the qualitative detection of the presence or absence of D-dimer at a level of not less than an optionally established concentration; the method is used in a way such that positive results are produced if the presence of D-dimer at a level of not less than the cutoff value is detected, and negative results are produced if it is not detected, provided that the cutoff value (D-dimer concentration) described below is determined in advance.

Note that immunochemical methods intended to measure D-dimer concentration in blood, such as the above-described sandwich ELISA method and latex aggregation method, are already known; D-dimer assay reagents utilizing these methods are commercially available, as described below. By using these reagents, a measurement of D-dimer concentration in blood separated from a human can easily be performed.

In the present invention, the blood used as a sample may be any of whole blood, serum, and plasma, and these can be obtained as appropriate by treating blood drawn from a human by a conventional method.

The determination whether or not acute aortic dissection has developed can be made by comparing the D-dimer concentration in blood which is measured as described above with, for example, the mean D-dimer concentration in blood which is previously established for a large number of patients with acute aortic dissection, or with the distribution of measured values in a large number of patients. For example, as measured in an Example below, the mean D-dimer concentration in blood from patients with acute aortic dissection, including Stanford type A and Stanford type B, was 33.0 µg/mL.

The present invention also relates to a method of evaluating acute aortic dissection, which comprises determining whether or not Stanford type A acute aortic dissection has developed, on the basis of the D-dimer concentration in blood which is measured as described above, and to a method of evaluating acute aortic dissection, which comprises determining whether or not Stanford type B acute aortic dissection has developed, in the same manner.

The determination whether or not Stanford type A acute aortic dissection has developed, and the determination whether or not Stanford type B acute aortic dissection has developed, can be performed in the same manner as the above-described determination whether or not acute aortic dissection has developed.

For example, as measured in an Example below, the mean D-dimer concentration in blood from 18 patients with Stanford type A acute aortic dissection was 48.3 µg/mL, and the mean D-dimer concentration in blood from 14 patients with Stanford type B acute aortic dissection was 14.5 µg/mL (Example 1).

As stated above, the mean D-dimer concentration in blood from the patients with Stanford type A acute aortic dissection was more than 3 times higher than that for the patients with Stanford type B acute aortic dissection (Example 1). This finding demonstrates that the evaluation method of the present invention also makes it possible to determine whether developed acute aortic dissection is of Stanford type A or Stanford type B, on the basis of the D-dimer concentration in blood from a patient diagnosed as developing acute aortic dissection.

When performing the above-described evaluation method of the present invention, the evaluation of acute aortic dissection can be made more accurate by combining with other methods which are known ordinary tests for cardiovascular diseases, for example, chest plain roentgenography, ultrasonic tomography, CT scanning and the like.

Another mode of the present invention relates to a method of distinguishing between acute aortic dissection and acute myocardial infarction, which comprises measuring the D-dimer concentration in blood separated from a human with suspected acute aortic dissection and/or acute myocardial infarction, for example, a human having an episode of chest pain, and determining which disease has developed, whether acute aortic dissection or acute myocardial infarction, on the basis of the measured concentration.

Acute myocardial infarction, another subject disease to be distinguished, is a disease that the coronary artery, which controls the myocardium, is occluded and the myocardium under the control thereof causes necrosis. The World Health Organization (WHO) has presented diagnostic criteria to determine that a subject is developing acute myocardial infarction if he or she meets all the requirements: A) chest pain, B) an abnormal electrocardiogram, and C) an elevation of blood levels of myocardial leaking enzymes such as creatine kinase-MB and aspartate aminotransferase.

Acute myocardial infarction has recently been increasing dramatically with the westernization of dietary life in Japan. This disease develops suddenly with the major symptoms of severe chest pain and dyspnea, and follows a very rapid course with cardiac arrest occurring simultaneously with the onset in some cases depending on the size of the infarct lesion in the myocardium. Hence, patients often die in 1 to 2 hours after onset; making the quick and accurate diagnosis of this disease and taking appropriate measures corresponding to it are important to saving their lives. Violent chest pain, a symptom characteristic of acute myocardial infarction, is also called an episode of chest pain, as in acute aortic dissection, and may involve the back to show signs of chest back pain.

Acute aortic dissection and acute myocardial infarction are called as "the two major diseases with chest pain" since they share an episode of chest pain as a characteristic clinical symptom, as described above, and these two diseases are difficult to distinguish from each other by clinical symptoms. However, if acute aortic dissection and acute myocardial infarction are suspected from an episode of chest pain and the like in actual medical settings, it is highly critical to distinguish which has developed, and to distinguish whether the acute aortic dissection is of Stanford type A or Stanford type B, so as to ensure the appropriate treatment.

Since the both diseases are called as "the two major diseases with chest pain", as stated above, "a human having an episode of chest pain" is the subject of measurement of D-dimer concentration in blood in the distinguishing method of the present invention.

As used herein, "a human having an episode of chest pain" means not only a human who had an episode of chest pain before blood separation, but also a human who is having an episode of chest pain at the time of blood separation.

Individual diseases can be distinguished by comparing the measured D-dimer concentration in blood with the pre-established mean D-dimer concentration in blood for a large number of patients with each disease, or with the distribution of measured values in a large number of patients, or the like, as in the aforementioned case of the evaluation method of the present invention.

For example, as measured in an Example below, the mean D-dimer concentration in blood from 18 patients with acute myocardial infarction was 0.39 µg/mL (Example 1).

Also, if a blood cutoff value of D-dimer concentration in blood cutoff value is previously established between acute aortic dissection and acute myocardial infarction, it is possible to determine that acute aortic dissection has developed if the measured D-dimer concentration is not lower than the aforementioned cutoff value, and that acute myocardial infarction, rather than acute aortic dissection, has developed if the measured D-dimer concentration is lower than the aforementioned cutoff value.

"A cutoff value" means a value that meets both the requirements for high diagnostic sensitivity (true positive rate) and high diagnostic specificity (true negative rate) when diseases are distinguished on the basis of the cutoff value as the criterion. In the present invention, the D-dimer concentration in blood, which shows high positive rates (not less than the cutoff value) in patients who have developed acute aortic dissection and shows high negative rates (less than the cutoff value) in patients who have developed acute myocardial infarction, can be used as a cutoff value.

The method of calculating a cutoff value is well known in the relevant field. For example, the D-dimer concentrations in blood from a large number of patients with acute aortic dissection or acute myocardial infarction are measured, and the diagnostic sensitivity and diagnostic specificity at each measured concentration are determined. Then, an ROC (Receiver Operating Characteristic) curve is generated on the basis of these measured values, and the D-dimer concentration for a diagnostic sensitivity and diagnostic specificity as close to 100% as possible is determined, and this value can be used as the cutoff value. Note that an ROC curve can also be generated using a commercially available analytical software program as shown in Examples below.

For example, the blood D-dimer cutoff values between acute aortic dissection and acute myocardial infarction calculated in Examples below were 0.7 µg/mL (Example 2) and 1.3 µg/mL (Examples 4 and 6). Therefore, for example, values of 0.7 to 1.3 µg/mL can also be used as cutoff values, as well as the above-described values. Such cutoff value does not have a particular value, but does change depending on the patient population used for D-dimer detection.

Note that even when the same sample is analyzed, the measured value of D-dimer concentration may differ depending on the measuring method and reagent used; therefore, the cutoff value must be established for each measuring method and reagent used for measuring the D-dimer.

Because D-dimer concentration in bloods differ widely between patients with Stanford type A acute aortic dissection and patients with Stanford type B acute aortic dissection, as stated above, it is also possible to determine which disease has developed, whether Stanford type A acute aortic dissection, Stanford type B acute aortic dissection, or acute myocardial infarction, in another embodiment included in the distinguishing method of the present invention.

When performing the above-described distinguishing method of the present invention, distinguishing between individual diseases can be made more accurate by combining with other methods which are known ordinary tests for cardiovascular diseases, for example, chest plain roentgenography, ultrasonic tomography, CT scanning and the like.

The present invention also relates to a reagent for evaluating acute aortic dissection, which is used to perform an immunochemical method out of the above-described evaluation methods of the present invention, and, which comprises an antibody that recognizes a D-dimer.

This evaluation reagent can preferably be used in cases where acute aortic dissection is evaluated by measuring a D-dimer concentration in blood by an immunochemical method, and is intended to accomplish the same objective as the evaluation method.

The anti-D-dimer antibody contained in the evaluation reagent of the present invention may be any of a polyclonal antibody and a monoclonal antibody, as long as it recognizes a D-dimer present in blood separated from a human, and has specific immunoreactivity to a D-dimer. Of the two types of antibodies, a monoclonal antibody is preferred in terms of stable supply of antibody, and also in terms of high specificity for D-dimer and homogeneity.

Such an anti-D-dimer antibody can be produced by a known means, and is contained in the evaluation reagent of the present invention in a free state, in a labeled state, or in a solid-immobilized state.

A polyclonal antibody can be produced by immunizing an animal such as a mouse, rat, or rabbit with a D-dimer separated and purified from human blood by a conventional method, along with an appropriate adjuvant, drawing blood, and subjecting the blood to a known treatment.

A monoclonal antibody can be produced by collecting splenocytes from an animal immunized as described above, fusing the splenocytes with myeloma cells by the method of Milstein et al., performing antibody-producing cell screening and cloning and the like, establishing a cell line that produces an anti-D-dimer antibody, and culturing the cell line. Here, the D-dimer used as an immune antigen is not necessarily be a naturally occurring D-dimer presented in human blood, and may be a recombinant D-dimer obtained by a gene engineering technique or an equivalent thereto (fragment) having the same effect.

When the sandwich ELISA method is adopted for the evaluation reagent of the present invention, the thus-obtained anti-D-dimer antibody is contained in the reagent in the form of a solid-immobilized anti-D-dimer antibody and an enzyme-labeled anti-D-dimer antibody.

A solid-immobilized anti-D-dimer antibody can be produced by binding an anti-D-dimer antibody obtained as described above to a solid phase (for example, microplate wells and plastic beads). Binding to the solid phase can be achieved usually by dissolving the antibody in an appropriate buffer solution such as a citrate buffer solution, and contacting the solid phase surface and the antibody solution for an appropriate time (1 - 2 days).

Furthermore, it is common practice to contact a phosphate buffer solution of bovine serum albumin (BSA), bovine milk protein or the like with the solid phase, and block the solid phase surface portion not coated by the antibody with the aforementioned BSA, bovine milk protein or the like, so as to suppress non-specific adsorption and non-specific reactions.

An enzyme-labeled anti-D-dimer antibody can be produced by binding (labeling) an anti-D-dimer antibody that recognizes an epitope different from that of the above-described solid-immobilized antibody and an enzyme. As the enzyme for labeling the antibody, alkaline phosphatase, glucose oxidase, peroxidase, β-galactosidase and the like can be mentioned. Binding of these enzymes and an anti-D-dimer antibody can be achieved by a method known per se, for example, the glutaraldehyde method, the maleimide method and the like.

When the aforementioned avidin-biotin reaction is utilized in the ELISA method, the biotin-labeled anti-D-dimer antibody contained in the reagent for evaluating acute aortic dissection can be produced by using, for example, a commercially available biotin-labeling kit.

When performing the sandwich ELISA method, a standard substance, a washing solution, enzyme activity evaluation reagents (substrate, substrate solvent, reaction stop solution and the like), enzyme-labeled streptoavidin (in cases where an avidin-biotin reaction is utilized) and the like are used as necessary, in addition to the aforementioned anti-D-dimer antibody. Therefore, the reagent for evaluating acute aortic dissection of the present invention may be in the form of a kit comprising these as constituent reagents, in addition to the anti-D-dimer antibody.

As the aforementioned substrate, an appropriate one according to the labeled enzyme selected is chosen. For example, when peroxidase is selected as the enzyme, o-phenylenediamine (OPD), tetramethylbenzidine (TMB) and the like are used; when alkaline phosphatase is selected, p-nitrophenyl phosphate (PNPP) and the like are used.

For the reaction stop solution and substrate solvent, conventionally known ones can be used as appropriate without limitation according to the labeled enzyme selected.

Note that a large number of D-dimer assay reagents and kits based on the sandwich ELISA method are commercially available (for example, Boehringer Mannheim "Asserachrom D-dimer" (trade name), Fujirebio "Dimer Test EIA" (trade name) and the like), and these reagents and the like can also be used as the reagent for evaluating acute aortic dissection of the present invention.

When the latex aggregation method is adopted for the evaluation reagent of the present invention, the anti-D-dimer antibody is contained in the reagent in the form of a latex-sensitized anti-D-dimer antibody. Sensitization (binding) of latex particles and an anti-D-dimer antibody can be achieved by a method known in the relevant field, for example, the chemical bonding method (a method using carbodiimide, glutaraldehyde or the like as a cross-linking agent) and the physical adsorption method.

When performing the latex aggregation method, a dilution-stabilizing buffer solution, a standard antigen and the like are used as necessary, in addition to the above-described latex-sensitized antibody. Therefore, the evaluation reagent of the present invention may be embodied in the form of a kit for evaluating acute aortic dissection, which comprises these as constituent reagents, in addition to the latex-sensitized anti-D-dimer antibody.

Note that a large number of D-dimer assay reagents and kits based on the latex aggregation method are commercially available (for example, DIA-IATRON "LPIA ACE D-D-dimer" (trade name), Nippon Roche "COAGUSOL D-dimer" (trade name) and the like), and these reagents and the like can also be used as the reagent for evaluating acute aortic dissection of the present invention.

When immunochromatography method is adopted for the evaluation reagent of the present invention, the anti-D-dimer antibody is contained in the reagent in the form of a solid-immobilized anti-D-dimer antibody and a labeled anti-D-dimer antibody. As the marker in the labeled anti-D-dimer antibody, ones known in the relevant field can be used as appropriate; as examples of such markers, gold colloid particles can be mentioned.

Such an evaluation reagent of the present invention based on immunochromatography can be produced with reference to the method described in Watanabe et al., Clinical Biochemistry 34 (2001) 257-263.

The evaluation reagent of the present invention is provided in the form of a commercial package in actual medical settings. Such a commercial package comprises the above-described evaluation reagent, which has the statement thereon that the evaluation reagent can be used, or should be used, for the purpose of evaluating acute aortic dissection, and/or has a printed matter on the evaluation reagent bearing the same statement therein.

The present invention also relates to a reagent for distinguishing between acute aortic dissection and acute myocardial infarction, which is used to perform an immunochemical method out of the above-described distinguishing methods between acute aortic dissection and acute myocardial infarction in the present invention, and which comprises an antibody that recognizes the D-dimer.

The reagent assumes the same configuration as the aforementioned evaluation reagent, can be preferably used in cases where the D-dimer concentration in blood is measured by an immunochemical method, and it is determined which disease has developed, whether acute aortic dissection or acute myocardial infarction, and is intended to accomplish the same object as the distinguishing method.

The above-described distinguishing reagent is provided in the form of a commercial package in actual medical settings. Such a commercial package comprises the above-described distinguishing reagent and has the statement thereon that the distinguishing reagent can be used, or should be used, for the purpose of distinguishing between acute aortic dissection and acute myocardial infarction, and/or comprises a printed matter on the evaluation reagent bearing the same statement therein.

### Examples

The present invention is hereinafter described in more detail by means of the following Examples, which, however, are not deemed to limit the scope of the invention.

### Example 1: Detection of D-dimer in blood from patients with acute aortic dissection (comparison with patients with acute myocardial infarction)

Of patients who visited a hospital with a complaint of chest pain (including chest back pain), those having the established diagnosis of acute aortic dissection (32 cases in total, consisting of 18 cases with Stanford type A and 14 cases with Stanford type B) and those having the established diagnosis of acute myocardial infarction (18 cases) had their blood drawn at the time of visit, and the D-dimer concentrations in the blood samples were measured as described below to obtain the results.

Note that informed consent was obtained from these patients.

### (1) Measurement of D-dimer concentrations

D-dimer concentrations were measured using "STA Liatest D-dimer" (trade name, manufactured by Roche Diagnostics K.K.), a D-dimer assay reagent based on the latex aggregation method as the measurement principle, as described below. Note that the reference value for this reagent (the upper limit for healthy people serving as a criterion for common determination of abnormalities in the blood coagulation/fibrinolytic system) has been established at 0.4 µg/mL.

1.8 mL of whole blood was drawn from a vein of each patient into a commercially available sodium citrate blood drawing tube (INSEPACK-C for coagulation testing [Sekisui Chemical Co., Ltd.], 0.2 mL of 3.13% sodium citrate contained). Subsequently, the blood drawing tube was gently inverted to mix the contents, and centrifuged (3000 rpm) at room temperature for 5 minutes. After centrifugation, the blood drawing tube as is was set to the automated D-dimer measuring equipment STA (manufactured by Roche Diagnostics K.K.) and analyzed. Regarding analytical conditions, 50 µL of blood sample, 100 µL of buffer solution (reagent 1 attached to the above-described D-dimer assay reagent; 11.5 mg/mL of tris(hydroxymethyl)aminomethane, 1 mg/mL of sodium azide, 23.5 mg/mL of NaCl, 0.95 mg/mL of methyl para-oxybenzoate [antiseptic]), 50 µL of diluent for STA instruments for the above-described D-dimer assay reagent, and 150 µL of latex-sensitized anti-D-dimer antibody solution (reagent 2 attached to the above-described D-dimer assay reagent; 55 µg/mL of antihuman D-dimer mouse monoclonal antibody, 0.65 mg/mL of latex, 3 mg/mL of bovine serum albumin, 0.95 mg/mL of methyl para-oxybenzoate [antiseptic], 1 mg/mL of sodium azide) were automatically added, and a measurement was performed with an analytical time of 240 seconds and a reaction temperature of 37°C. The calibration curve range for this assay reagent is 0.2 to 4.0 µg/mL of D-dimer concentration; samples showing high values exceeding this concentration range were subjected to reanalysis. The samples with high values were diluted with the diluent for STA instruments at a pre-defined dilution rate in advance, and again applied to the STA equipment to measure the D-dimer concentration in the sample. The measured D-dimer values were computer-processed and managed on line.

### (2) Measurement results

The measurement results are shown in Table 1 below; the D-dimer concentration in blood from patients developing acute aortic dissection was much higher than that in patients developing acute myocardial infarction. By Welch t-test, a statistically significant difference was observed between the two groups. Also in comparisons of the acute myocardial infarction group and the Stanford type A group, and of the acute myocardial infarction group and the Stanford type B group, a statistically significant difference was observed.

Note that a statistically significant difference was also observed between the Stanford type A group and the type B group.

**Table 1. D-dimer Concentrations in Blood from Patients with Acute Aortic Dissection and Patients with Acute Myocardial Infarction**

| Patient | Number of cases | D-dimer concentration (µg/mL) (Mean ± standard deviation) |
|---|---|---|
| Acute aortic dissection (Stanford types A and B) | 32 | 33.0±59.8** |
| Stanford type A | 18 | 48.3±77.4* |
| Stanford type B | 14 | 14.5±15.2** |
| Acute myocardial infarction | 18 | 0.39±0.39 |

| | | |
|---|---|---|
| *: Significantly different from the acute myocardial infarction group at a significance level of 5%. | | |
| **: Significantly different from the acute myocardial infarction group at a significance level of 1%. | | |

### Example 2: Establishing a cutoff value (in cases where acute myocardial infarction was the control disease to be distinguished)

Of patients who visited a hospital with a complaint of chest pain (including chest back pain), those having the established diagnosis of acute aortic dissection (60 cases) and those having the established diagnosis of acute myocardial infarction (37 cases) had their blood drawn, and D-dimer concentrations were measured in the same manner as Example 1. Note that informed consent was obtained from these cases. Note that the cases of this Example include the cases of Example 1 above.

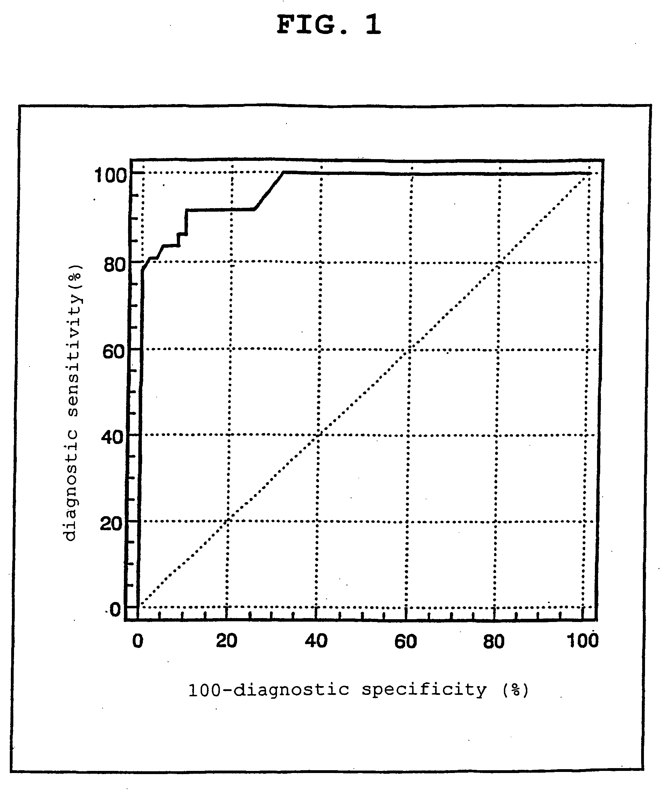

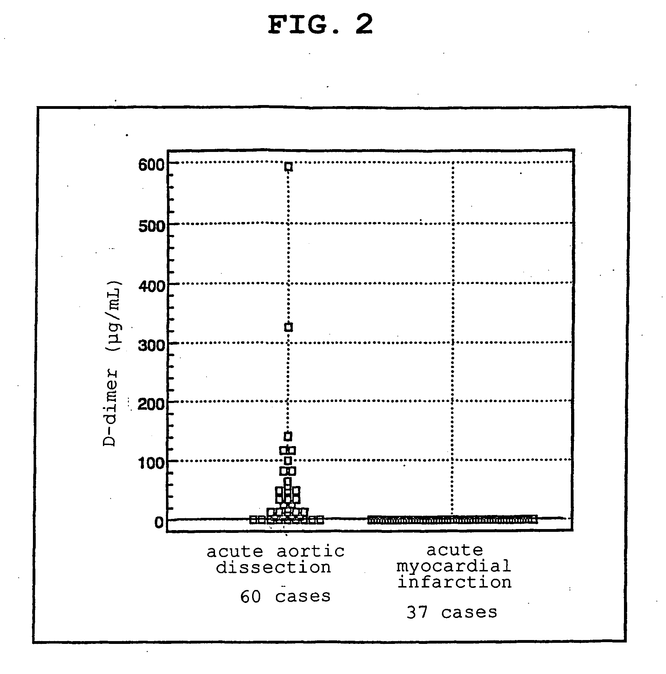

For the measured values obtained, ROC curve analysis was performed using the ROC analytical software program "MedCalc" (trade name, Med Calc Software Company [Belgium]), and a D-dimer cutoff value that makes it possible to distinguish between acute aortic dissection and acute myocardial infarction was calculated. The calculated value was 0.7 µg/mL. FIG. 1 shows the ROC curve used in this analysis, and FIG. 2 shows the distribution of measured values. Note that the cutoff value of 0.7 µg/mL had a diagnostic sensitivity (ratio of positive cases to all cases with acute aortic dissection) of 91.9% and a diagnostic specificity (ratio of negative cases to all cases with acute myocardial infarction) of 90.0%, showing very high accuracy. Hence, the D-dimer concentration in blood of 0.7 µg/mL proved to be a cutoff value that makes it possible to clearly distinguishing between acute aortic dissection and acute myocardial infarction, which is in the greatest need for distinguishing of similar disease from acute aortic dissection.

The area under curve (AUC) for the ROC curve shown in FIG. 1 was 0.968, a value very close to 1. This means that D-dimer concentration in bloods serve as a useful marker in distinguishing between acute aortic dissection and acute myocardial infarction.

### Example 3: Applying the cutoff value determined in Example 2

To verify the diagnostic accuracy of the cutoff value (0.7 µg/mL) for distinguishing between acute aortic dissection and acute myocardial infarction established in Example 2, the positive rates (ratios of cases showing a value not lower than the cutoff value) in the patients with acute aortic dissection and the patients with acute myocardial infarction, which are cases in Example 1, were calculated and are shown in Table 2 below.

**Table 2. Positive rates in Patients with Acute Aortic Dissection and Patients with Acute Myocardial Infarction**

| Patient | Number of cases of cases | Number of positive cases | Positive rate (%) |
|---|---|---|---|
| Acute aortic dissection (Stanford types A and B) | 32 | 29 | 90.6 |
| Stanford type A | 18 | 17 | 94.4 |
| Stanford type B | 14 | 12 | 85.7 |
| Acute myocardial infarction | 18 | 3 | 16.7 |

Cutoff value: 0.7 µg/mL

As shown in Table 2 above, when the cutoff value was established at 0.7 µg/mL in the cases of Example 1, patients with acute aortic dissection showed a very high positive rate (that is, the diagnostic sensitivity was 90.6%). Of the 18 cases of the patients with acute myocardial infarction, 15 cases had a value lower than the cutoff value, the diagnostic specificity reaching 83.3% (100% - 16.7%). This means that 0.7 µg/mL serves as a cutoff value that makes it possible to clearly distinguish between acute aortic dissection and acute myocardial infarction.

### Example 4: Establishing a cutoff value in the cases of Example 1

For the cases of Example 1, a cutoff value that makes it possible to distinguish between acute aortic dissection and acute myocardial infarction was determined in the same manner as Example 2. The calculated cutoff value was 1.3 µg/mL. The area under curve for the ROC curve was 0.960, and this cutoff value had a diagnostic sensitivity of 87.9% and a diagnostic specificity of 100%, showing very high diagnostic accuracy.

### Example 5: Measurement of the D-dimer concentrations in blood from patients developing acute aortic dissection (Stanford type A and Stanford type B) (comparison with patients with acute myocardial infarction)

Of patients who visited a hospital with a complaint of chest pain (including chest back pain), those having the established diagnosis of acute aortic dissection (66 cases in total, consisting of 39 cases with Stanford type A and 27 cases with Stanford type B) and those having the established diagnosis of acute myocardial infarction (62 cases) had their blood drawn at the time of hospital visit, and the D-dimer concentrations in the blood samples were measured as described below to obtain the results. The patients of this Example include the patients of Example 2 above.

Note that informed consent was obtained from these patients.

### (1) Measurement of D-dimer concentrations

D-dimer concentrations were measured in the same manner as Example 1 using "STA Liatest D-dimer" (trade name, manufactured by Roche Diagnostics K.K.), a D-dimer assay reagent based on the latex aggregation method as the measurement principle.

### (2) Measurement results

The measurement results are shown in Table 3 below.

**Table 3. D-dimer Concentrations in Blood from Patients with Acute Aortic Dissection (Stanford types A and B) and Patients with Acute Myocardial Infarction**

| Patient | Number of cases | D-dimer concentration (µg/mL) (Mean ± standard deviation) |
|---|---|---|
| Acute aortic dissection | 66 | 33.0±56.7** |
| Stanford type A | 39 | 42.1±64.0** |
| Stanford type B | 27 | 19.9±41.7** |
| Acute myocardial infarction | 62 | 1.7±5.7 |

| | | |
|---|---|---|
| **: Significantly different from the acute myocardial infarction group at a significance level of 1%. | | |

- As shown in Table 3 above, the D-dimer concentration in blood from patients developing acute aortic dissection was much higher than that in patients with acute myocardial infarction, who are known to have higher D-dimer concentration in bloods than healthy people. By Mann-Whitney U-test, a statistically significant difference was observed between the two groups.

When the patients with Stanford type A acute aortic dissection and patients with Stanford type B acute aortic dissection were compared, the D-dimer concentration was more than 2 times higher for Stanford type A than for Stanford type B, as shown in Table 3 above.

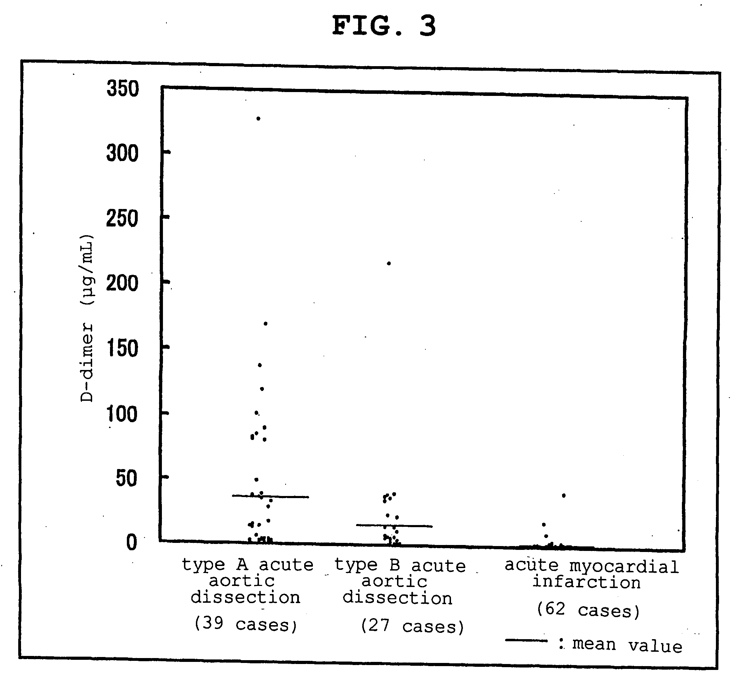

FIG. 3 shows the distribution of measured values of D-dimer concentration in bloods in patients with each disease.

### Example 6: Distinguishing between acute aortic dissection and acute myocardial infarction by cutoff value

### (1) Establishing a cutoff value between acute aortic dissection and acute myocardial infarction

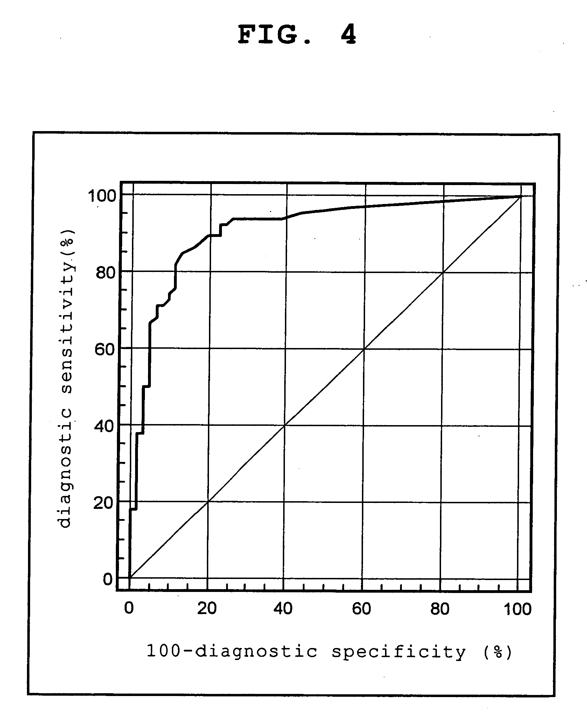

For the measured values obtained in Example 5, ROC curve analysis was performed using the ROC analytical software program "MedCalc" (trade name, Med Calc Software Company [Belgium]), and a D-dimer cutoff value was established between acute aortic dissection and acute myocardial infarction. The value calculated from the ROC curve (FIG. 4) was 1.3 µg/mL.

The area under curve (AUC) for the ROC curve shown in FIG. 4 was 0.911, a value very close to 1. This means that D-dimer concentration in bloods serve as a useful marker for distinguishing between acute aortic dissection and acute myocardial infarction.

### (2) Distinguishing between acute aortic dissection and acute myocardial infarction by the cutoff value

An attempt was made to distinguish between the cases with acute aortic dissection and cases with acute myocardial infarction described in Example 5, using the cutoff value determined in (1) above.

As a result, when the cutoff value was established as 1.3 µg/mL, the diagnostic sensitivity and diagnostic specificity were shown in Table 4 below.

In Table 4, diagnostic sensitivity indicates the ratio of positive cases to all cases with acute aortic dissection, and diagnostic specificity indicates the ratio of negative cases to all cases with acute myocardial infarction.

**Table 4. Distinguishing between Acute Aortic Dissection and Acute Myocardial Infarction by the Aforementioned Cutoff Value**

| Subject disease | Diagnostic criteria (D-dimer concentration in blood) | Diagnostic sensitivity | Diagnostic specificity |
|---|---|---|---|
| Acute aortic dissection | Positive (not less than 1.3 µg/mL) | 84.8% (56/66) | 87.1% (54/62) |
| Acute myocardial infarction | Negative (less than 1.3 µg/mL) | | |

As shown in Table 4 above, when the cases with acute aortic dissection and cases with acute myocardial infarction of Example 5 were distinguished using the cutoff value established in (1) above, diagnostic sensitivity was 84.8% and diagnostic specificity was 84.8%, showing very high values.

This result means that the D-dimer concentration in blood of 1.3 µg/mL serves as a cutoff value that makes it possible to highly accurately distinguish between acute aortic dissection and acute myocardial infarction.

### Industrial Applicability

By using the evaluation method and evaluation reagent of the present invention, it is possible to evaluate acute aortic dissection among diseases of similar clinical symptoms, such as acute myocardial infarction, and it is further possible to determine whether the developed acute aortic dissection is of Stanford type A or of Stanford type B. Physicians can give the appropriate treatment for each disease on the basis of the results of this evaluation and the like.

The present application is based on Japanese patent application no. 2003-165456 filed in Japan (filing date: June 10, 2003) and its content is herein incorporated by reference.

## Claims

1. A method of evaluating acute aortic dissection, which comprises measuring a D-dimer concentration in blood separated from a human, and determining whether or not acute aortic dissection has developed, on the basis of the measured concentration.

2. A method of evaluating acute aortic dissection, which comprises measuring a D-dimer concentration in blood separated from a human, and determining whether or not Stanford type A acute aortic dissection has developed, on the basis of the measured concentration.

3. A method of evaluating acute aortic dissection, which comprises measuring a D-dimer concentration in blood separated from a human, and determining whether or not Stanford type B acute aortic dissection has developed, on the basis of the measured concentration.

4. A method of evaluating acute aortic dissection, which comprises measuring a D-dimer concentration in blood separated from a human developing acute aortic dissection, and determining whether the developed acute aortic dissection is Stanford type A acute aortic dissection or Stanford type B acute aortic dissection, on the basis of the measured concentration.

5. A method of distinguishing between acute aortic dissection and acute myocardial infarction, which comprises measuring a D-dimer concentration in blood separated from a human having an episode of chest pain, and determining which disease has developed whether acute aortic dissection or acute myocardial infarction, on the basis of the measured concentration.

6. The distinguishing method of claim 5, which comprises comparing the measured D-dimer concentration in blood and a D-dimer cutoff value in blood which is pre-established between acute aortic dissection and acute myocardial infarction, and determining that acute aortic dissection has developed if said concentration is not lower than said cutoff value, and acute myocardial infarction has developed if said concentration is lower than said cutoff value.

7. A method of distinguishing between acute aortic dissection and acute myocardial infarction, which comprises measuring a D-dimer concentration in blood separated from a human having an episode of chest pain, and determining which disease has developed whether Stanford type A acute aortic dissection, Stanford type B acute aortic dissection, or acute myocardial infarction, on the basis of the measured concentration.

8. The evaluation method for acute aortic dissection of any of claims 1 to 4, wherein the measurement of D-dimer concentration in blood is performed by an immunochemical method.

9. The evaluation method of claim 8, wherein the immunochemical method is an enzyme immunochemical method, a latex aggregation method, or an immunochromatography method.

10. The method of distinguishing between acute aortic dissection and acute myocardial infarction of any of claims 5 to 7, wherein the measurement of D-dimer concentration in blood is performed by an immunochemical method.

11. The distinguishing method of claim 10, wherein the immunochemical method is an enzyme immunochemical method, a latex aggregation method, or an immunochromatography method.

12. A reagent for evaluating acute aortic dissection, which comprises an antibody that recognizes a D-dimer.

13. The evaluation reagent of claim 12, which is a reagent for evaluating Stanford type A acute aortic dissection.

14. The evaluation reagent of claim 12, which is a reagent for evaluating Stanford type B acute aortic dissection,

15. A reagent for distinguishing between Stanford type A acute aortic dissection and Stanford type B acute aortic dissection, which comprises an antibody that recognizes a D-dimer.

16. The reagent of any of claims 12 to 15, wherein the antibody is a monoclonal antibody.

17. A reagent for distinguishing between acute aortic dissection and acute myocardial infarction, which comprises an antibody that recognizes a D-dimer.

18. The distinguishing reagent of claim 17, which is a reagent for distinguishing between Stanford type A acute aortic dissection and acute myocardial infarction.

19. The distinguishing reagent of claim 17, which is a reagent for distinguishing between Stanford type B acute aortic dissection and acute myocardial infarction.

20. The distinguishing reagent of claim 17, which is a reagent for distinguishing among Stanford type A acute aortic dissection, Stanford type B acute aortic dissection, and acute myocardial infarction.

21. The distinguishing reagent of any of claims 17 to 20, wherein the antibody is a monoclonal antibody.

22. A commercial package comprising the reagent of any of claims 12 to 15 and a printed matter on the reagent, wherein the printed matter and/or the package bears the statement that the reagent can be used, or should be used, for the purpose of evaluating acute aortic dissection.

23. A commercial package comprising the distinguishing reagent of any of claims 17 to 20 and a printed matter on the reagent, wherein the printed matter and/or the package bears the statement that the reagent can be used, or should be used, for the purpose of distinguishing between acute aortic dissection and acute myocardial infarction.

24. Use of an antibody that recognizes a D-dimer, for producing a reagent for evaluating acute aortic dissection.

25. Use of an antibody that recognizes a D-dimer, for producing a reagent for distinguishing between acute aortic dissection and acute myocardial infarction.
